# EUROPEAN PATENT APPLICATION

(11) **EP 4 672 249 A1**
(43) Date of publication of application: **31.12.2025**
(21) Application number: 24764130.1
(22) Date of filing: 22.02.2024
(51) Int. Cl.: G16C 20/30, G16C 20/20, G16C 20/80

(54) **METHOD AND SYSTEM FOR SEPARATING BIOCHEMICAL MOLECULES**

(30) Priority: 27.02.2023 KR 20230026000
(71) Applicant: METEOR BIOTECH, CO. LTD., Seoul 08790 (KR)
(72) Inventor: KWON, Sunghoon, Seoul 06101 (KR); LEE, Chungwon, Seoul 08796 (KR); LEE, Su Min, Gwangmyeong-si, Gyeonggi-do 14349 (KR); KIM, Kyong Jun, Suwon-si, Gyeonggi-do 16229 (KR); JANG, Hae Wook, Seoul 08788 (KR)
(74) Representative: Prüfer & Partner mbB Patentanwälte · Rechtsanwälte
(86) International application number: PCT/KR2024/002293
(87) International publication number: WO 2024/181730

(57) **Abstract**

The present invention relates to a method and system for isolating biochemical molecules, and the method for isolating biochemical molecules may include the steps of: imaging a tissue sample to acquire a tissue image in the form of any one of a multi-channel fluorescence image and a visible light image; obtaining and analyzing detection results for each channel from the tissue image to extract spatial information of a target, or analyzing the tissue image through a learned neural network model to predict spatial information of a target; and adjusting the size and location of a isolation area in a biochemical molecule isolation device based on the spatial information of the target, and then physically isolating and retrieving the target from the tissue sample.

## Description

### [Technical Field]

The present invention relates to a method and system for isolating biochemical molecules, which enables cells for spatial omics to be automatically detected and then isolated and retrieved more quickly.

### [Background Art]

Spatial omics profiling technologies have recently enabled the deciphering of genetic molecules structurally related to pathology.

In tumor biology, tumors are not diseases caused solely by tumor cells, but rather complex diseases in which various cells, such as immune cells, vascular structures and epidermal cells, form a community and develop the diseases.

Technologies for analyzing tumors have evolved from bulk sequencing and single-cell sequencing to spatial omics profiling technologies, and have had a significant impact on deciphering cancer mechanisms by questioning tumor heterogeneity, tumor microenvironment and spatial biomarkers.

Most spatial omics technologies focus on mapping the spatial omics landscape on a large scale, and introduce spatially barcoded capture probes or fluorescently labeled target probes to locate genetic molecules.

These technologies vary in the depth and scalability of information depending on the purpose of the spatial analysis technique, wherein spatial heterogeneity and spatial topography of constituted cell types can be found due to the relatively low depth, and deeper omics information is required to effectively address target molecules for therapy or diagnosis.

To meet these requirements, region of interest (ROI)-based spatial technologies isolate target regions and apply chemistry for higher-coverage omics data.

Conventional cell sorters include fluorescence-activated cell sorter (FACS), magnetic-activated cell sorting (MACS), laser capture microdissection (LCM), etc.

FACS or MACS use fluorescent particles or magnetic particles, respectively, to designate cells of interest in a solution of dissociated cells, but have the disadvantage that the spatial context is lost before the cells are sorted.

LCM can sort ROIs while maintaining spatial context, but has the disadvantage of requiring additional preparation time, such as focusing a UV beam to isolate the target, and being able to damage cells by dissecting the cells or melting IR-activated polymers to extrude the cells, using a UV laser.

### [Disclosure]

### [Technical Problem]

Accordingly, in order to solve the above problems, the present invention aims to provide a method and system for isolating biochemical molecules, which enables targets to be automatically detected and then isolated and retrieved more quickly while the spatial context of cells is preserved.

The object of the present invention is not limited to the above-mentioned object, and other objects that are not mentioned will be clearly understood by those skilled in the art to which the present invention pertains from the following description.

### [Technical Solution]

As a means for solving the above problems, the method for isolating biochemical molecules in a system for isolating biochemical molecules according to a first embodiment of the present invention includes imaging a tissue sample to acquire a tissue image in the form of any one of a multi-channel fluorescence image and a visible light image; obtaining and analyzing detection results for each channel from the tissue image to extract spatial information of a target, or analyzing the tissue image through a learned neural network model to predict spatial information of a target; and adjusting the size and location of an isolation area in a biochemical molecule isolation device based on the spatial information of the target, and then physically isolating and retrieving the target from the tissue sample.

In addition, it is characterized in that the target is any one of a cell, a group of two or more cells, a micro-region containing a cell, ribonucleic acid (RNA), deoxyribonucleic acid (DNA), an epigenome, an epitranscriptome, a metabolite, a cell organelle, a B cell receptor (BCR), a T cell receptor (TCR), an extracellular membrane protein, an extracellular vesicle, a carrier containing a biochemical molecule, and a hydrogel containing a biochemical molecule.

It is characterized in that the spatial information of the target may include at least one of information on location, size, shape, density, and staining pattern.

It is characterized in that the extracting of the spatial information of the target includes the following steps: acquiring a multi-channel fluorescence image; isolating fluorescence channels and then performing an object detection operation; comparing and analyzing object detection results for each fluorescence channel to filter the object detection results; and selecting at least one or more of the fluorescence channels in consideration of the type of the target, and extracting and providing the spatial information of the target from the object detection results of the selected fluorescence channel.

It is characterized in that, when the target is a cell, the filtering includes refining and extracting only the spatial information of the target, by acquiring object detection results for each fluorescence channel based on at least one of location, shape, size, density and fluorescence intensity and then filtering out duplicate objects and noise objects from the object detection result for each fluorescence channel.

It is characterized in that the neural network model is pre-trained on data mapping tissue images to target detection results, by repeatedly training on multiple datasets having a sample image as an input condition and a target detection probability as an output condition.

It is characterized in that the physically isolating and retrieving the target includes physically isolating the target through any one method of isolation using lasers, mechanical isolation through microdissection, isolation using high-frequency ultrasound, chemical isolation through the addition of specific chemicals or enzymes, biological isolation using antibodies specific to specific cell surface antigens, optical isolation using light, and thermal ablation using high temperatures.

As a means for solving the above problems, it is characterized in that the method for spatially designating biochemical molecules in a system for isolating biochemical molecules according to a second embodiment of the present invention includes the steps of: imaging a tissue sample to acquire a tissue image in the form of any one of a multi-channel fluorescence image and a visible light image; and obtaining and analyzing detection results for each channel from the tissue image to extract spatial information of a target, or analyzing the tissue image through a learned neural network model to acquire spatial information of a target, and then providing it to an external party.

It is characterized in that the target is any one of a cell, a group of two or more cells, a micro-region containing a cell, ribonucleic acid (RNA), deoxyribonucleic acid (DNA), an epigenome, an epitranscriptome, a metabolite, a cell organelle, a B cell receptor (BCR), a T cell receptor (TCR), an extracellular membrane protein, an extracellular vesicle, a carrier containing a biochemical molecule, and a hydrogel containing a biochemical molecule.

It is characterized in that the spatial information of the target may include at least one of information on location, size, shape, density, and staining pattern.

It is characterized in that the extracting of the spatial information of the target includes the steps of: acquiring a multi-channel fluorescence image; isolating fluorescence channels and then performing an object detection operation; comparing and analyzing object detection results for each fluorescence channel to filter the object detection results; and selecting at least one or more of the fluorescence channels in consideration of the type of the target, and extracting and providing the spatial information of the target from the object detection results of the selected fluorescence channel.

It is characterized in that the neural network model is pre-trained on data mapping tissue images to target detection results, by repeatedly training on multiple datasets having a sample image as an input condition and a target detection probability as an output condition.

As a means for solving the above problems, the method for providing spatial information of a target for a system for automatically isolating biochemical molecules according to a third embodiment of the present invention includes the steps of: acquiring a multi-channel fluorescence image; isolating fluorescence channels and then performing an object detection operation; comparing and analyzing object detection results for each fluorescence channel to filter out duplicate objects and noise objects; and selecting any one of the fluorescence channels in consideration of the type of the target to extract the spatial information of the target from the object detection results of the selected fluorescence channel and providing it to an external party.

As a means for solving the above problems, the method for providing spatial information of a target for a system for automatically isolating biochemical molecules according to a fourth embodiment of the present invention includes the steps of: pre-learning a neural network model through a plurality of training data having a sample image as an input condition and a target matching probability as an output condition; imaging a tissue sample to acquire a tissue image in the form of any one of a multi-channel fluorescence image and a visible light image; and analyzing the tissue image through the learned neural network model to extract spatial information of the target and providing it to an external party.

As a means for solving the above problems, the system for automatically isolating biochemical molecules according to a fifth embodiment of the present invention includes: a target automatic detection device for inputting a tissue image acquired in the form of any one of a multi-channel fluorescence image and a visible light image, and obtaining and analyzing detection results for each channel from the tissue image to extract spatial information of the target, or analyzing the tissue image through a learned neural network model to acquire spatial information of the target; and a biochemical molecule isolation device for acquiring the tissue image to provide it to the target automatic detection device, then adjusting the size and location of an isolation area based on the spatial information of the target fed back from the target automatic detection device, and then physically isolating and retrieving the target from a tissue sample.

### [Advantageous Effects]

The present invention enables spatial information of a target to be automatically detected using image processing technology, and a target isolation operation to be performed based on the same. As a result, it enables the time required for detection and isolation of the target to be dramatically reduced while the spatial context of the target is preserved.

### [Description of Drawings]

FIG. 1 is a drawing showing a system for automatically isolating biochemical molecules for spatial omics according to one embodiment of the present invention.
FIG. 2 is a drawing for explaining a device for isolating biochemical molecules according to one embodiment of the present invention.
FIG. 3 is a drawing for explaining the method for detecting a target based on a fluorescence image according to one embodiment of the present invention.
FIGS. 4 and 5 are drawings for explaining the object detection step for each channel according to one embodiment of the present invention.
FIG. 6 is a drawing for explaining the results of isolation and retrieval of a target according to one embodiment of the present invention.
FIG. 7 is a drawing for explaining an image stitching method of fluorescence images according to one embodiment of the present invention.
FIG. 8 is a drawing for explaining a method for detecting a target based on a neural network according to another embodiment of the present invention.
FIG. 9 is a drawing for explaining a method for generating microparticles according to another embodiment of the present invention.
FIG. 10 is a drawing for explaining a data augmentation method according to another embodiment of the present invention.
FIG. 11 is a drawing for explaining a neural network model according to another embodiment of the present invention.

### [Best Mode]

Hereinafter, preferred embodiments will be described in detail with reference to the accompanying drawings so that those skilled in the art to which the present invention pertains can easily practice the present invention. However, in describing preferred embodiments of the present invention in detail, if it is determined that the specific description of the related known functions or configurations may unnecessarily obscure the gist of the present invention, the detailed description thereof will be omitted. In addition, for parts that have similar functions and actions, the same symbols will be used throughout the drawings.

In addition, throughout the specification, when a part is said to be "connected" to another part, this includes not only cases where they are "directly connected," but also cases where they are "indirectly connected" by placing other elements therebetween. In addition, to "comprise or include" a component means to further comprise or include, but not to exclude, other components, unless otherwise specifically stated.

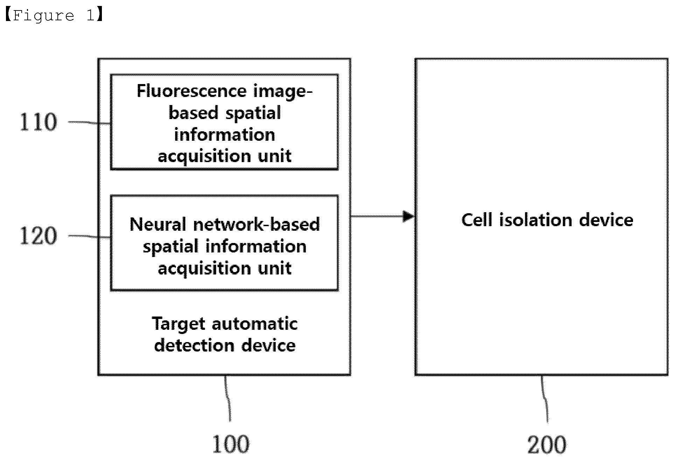

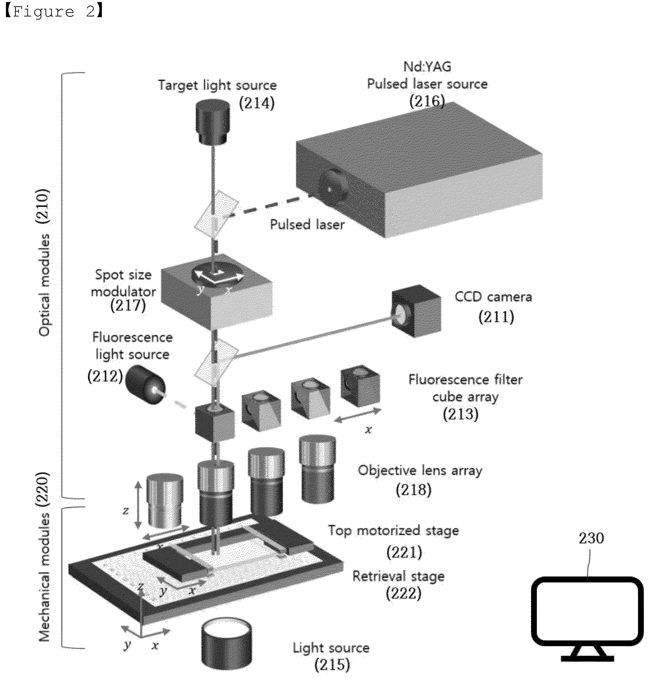

FIGS. 1 and 2 are drawings showing the system for automatically isolating biochemical molecules for spatial omics according to one embodiment of the present invention.

Referring to FIGS. 1 and 2, the device of the present invention is comprised of: a target automatic detection device 100 for inputting a tissue image acquired in the form of any one of a multi-channel fluorescence image and a visible light image, and obtaining and analyzing detection results for each channel from the tissue image to extract spatial information of the target, or analyzing the tissue image through a learned neural network model to acquire spatial information of the target; and a biochemical molecule isolation device 200 for acquiring the tissue image to provide it to the target automatic detection device 100, then adjusting the size and location of an isolation area based on the spatial information of the target fed back from the target automatic detection device 100, and then physically isolating and retrieving the target from a tissue sample.

The target automatic detection device 100 is equipped with a fluorescence image-based target detection unit 110 and a neural network-based target detection unit 120, so as to automatically extract spatial information of the target in two ways depending on whether or not the tissue sample is stained.

The fluorescence image-based target detection unit 110 extracts spatial information based on a stained tissue sample, which acquires a multi-channel fluorescence image of the tissue sample to acquire object detection results for each channel, and then compares and analyzes the object detection results for each channel to filter out detected objects. In addition, depending on the type of target, any one of the object detection results for each channel is selected and used to extract and provide spatial information of the target.

The neural network-based target detection unit 120 extracts spatial information based on an unstained tissue sample, which enables the correlation between the tissue image and the target detection results to utilize a learned neural network model. That is, after the tissue image is acquired, the image is analyzed through a neural network model to predict and provide spatial information of the target.

In this case, the target may be, but is not limited to, any one of a cell, ribonucleic acid (RNA), deoxyribonucleic acid (DNA), an epigenome, an epitranscriptome, a metabolite, a cell organelle, a B cell receptor (BCR), a T cell receptor (TCR), an extracellular membrane protein, and an extracellular vesicle.

In addition, the spatial information of the target may include, but is not limited to, location, size, shape, density, and staining pattern.

The biochemical molecule isolation device 200 is comprised of an optical module 210, a mechanical module 220, and a processor 230.

The optical module 210 includes a CCD camera 211 for imaging a tissue sample, a fluorescence light source 212 for irradiating a fluorescence light source into a laser light path, a filter cube array 213 including a plurality of fluorescence filters for expanding the range of a stained sample, a target light source 214 for illuminating the upper side of a target region, a light source 215 for illuminating the lower side of the target region, a Nd:YAG nanosecond pulsed laser source 216 for irradiating an NIR laser (λ = 1064 nm, pulse = 6 nanoseconds) for ablating a target through a fixed laser light path, a spot size modulator 217 for controlling the laser spot size depending on the slit size adjusted by the x-axis and the y-axis and the magnification of the objective lens, an objective lens 218 having a long working distance for focusing the laser spot and providing a sufficient working space for sorting a tissue sample, etc.

The mechanical module 220 is comprised of two motorized stages with a precision of less than a micrometer, i.e., a sample stage 221 for fixing the location of the ITO glass on which a tissue sample is placed, and a retrieval stage 222 for arranging a plurality of PCR tubes to be used as wells to retrieve cells isolated from the tissue sample.

In this case, the sample stage 221 focuses the real-time image through z-axis control under the assumption that the laser path is fixed, and aims at the target through x-axis and y-axis control.

If the tissue sample is stained, the processor 230 enables a multi-channel fluorescence image to be acquired through the filter cube array 213 and spatial information of the target to be acquired through the fluorescence image-based target detection unit 110, and if the tissue sample is not stained, it enables a visible light image to be acquired without using the filter cube array 213 and spatial information of the target to be acquired through the neural network-based target detection unit 120.

In addition, if the spatial information of the target is acquired, the laser spot size is controlled through the slit size of the spot size modulator 217 and the magnification of the objective lens 218 depending on the size of the target, and the location of the sample stage 221 is controlled depending on the location of the target so that the laser can be irradiated only to the target.

In addition, the location of the retrieval stage 222 is controlled so that the target isolated from the tissue sample can be isolated and retrieved through each well of the retrieval stage 222.

That is, the present invention enables both the operation of extracting spatial information of a target and the operation of isolating the target by irradiating a laser depending on the spatial information to be performed automatically, thereby minimizing the time and effort required for isolation of the target.

In addition, although the above description only explains cases where a target is physically isolated from a tissue sample using a laser, it is natural that various methods may be applied, such as mechanical isolation through microdissection, isolation using high-frequency ultrasound, chemical isolation through the addition of specific chemicals or enzymes, biological isolation using antibodies specific to specific cell surface antigens, optical isolation using light, and thermal ablation using high temperatures, if necessary.

Hereinafter, a method for detecting a target and acquiring and providing spatial information thereof by the system for automatically isolating biochemical molecules of the present invention will be described in more detail.

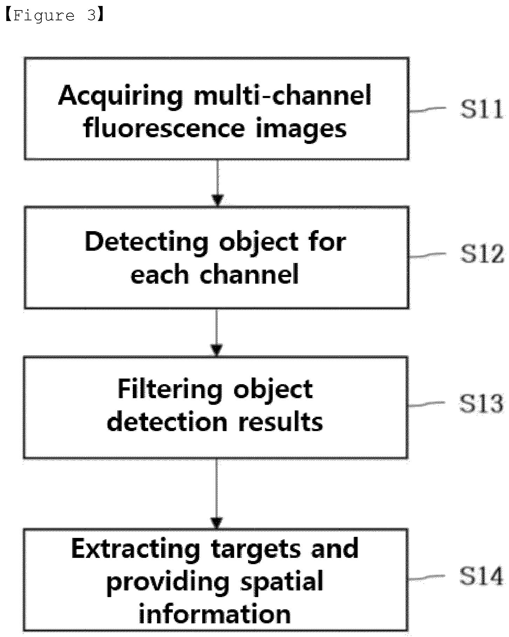

FIGS. 3 to 7 are drawings for explaining the method for detecting a target based on a fluorescence image according to one embodiment of the present invention.

As shown in FIG. 3, the method for acquiring the fluorescence image-based spatial information of the present invention is comprised of a step of acquiring multi-channel fluorescence image (S11), a step of detecting object for each channel (S12), a step of filtering object detection results (S13), and a step of extracting a target and providing spatial information (S14).

Hereinafter, for convenience of explanation, a multi-channel fluorescence image is comprised of fluorescence channels of DAPI, FITC and Cy5, and the case of detecting cancer cells as targets among nuclei, white blood cells and cancer cells will be described as an example.

### Step of acquiring multi-channel fluorescence images (S11)

After the tissue sample is stained according to a known technology such as H&E staining and immunostaining, multi-channel fluorescence images having fluorescence channels of DAPI, FITC and Cy5 are acquired through a filter cube array (213).

### Step of object detection for each channel (S12)

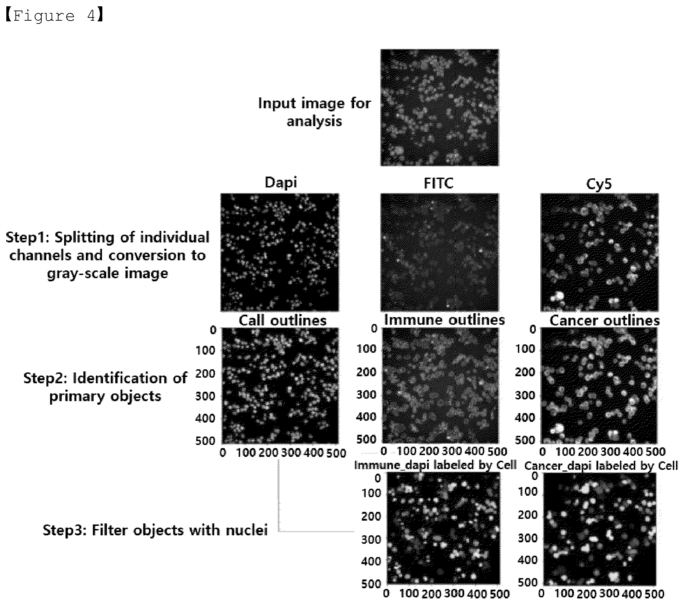

After the multi-channel fluorescence images are isolated into images of each fluorescence channel of DAPI, FITC and Cy5 as shown in FIG. 4, the images of each fluorescence channel are converted into grayscale images (Step 1).

In addition, using the open source tool Cell Profiler, nuclei are detected in the DAPI channel image, and circular objects likely to be cells are detected based on size, shape and color in the FITC channel image and Cy5 channel image, respectively (Step 2).

In addition, considering that cell lines such as white blood cells and cancer cells have the characteristic of having a nucleus, the nucleus detection results of the DAPI channel image and the circular object detection results of the FITC channel image are compared to detect only circular objects that exist in the same location as the nucleus, as white blood cells. In addition, by the same principle, the nucleus detection results of the DAPI channel image and the circular object detection results of the Cy5 channel image are compared to detect only circular objects that exist in the same location as the nucleus, as cancer cells (Step 3).

### Step of filtering object detection results (S13)

However, there is a problem that different cell lines may be photographed overlapping in images of specific fluorescence channels. For example, unlike the Cy5 channel, which only has fluorophores attached to cancer cells, the FITC channel has the problem of having fluorophores attached to cancer cells as well as fluorophores attached to white blood cells (especially anti-CD45). The FITC channel with an emission wavelength of 530 nm is known to overlap with the natural fluorescence emitted from cells.

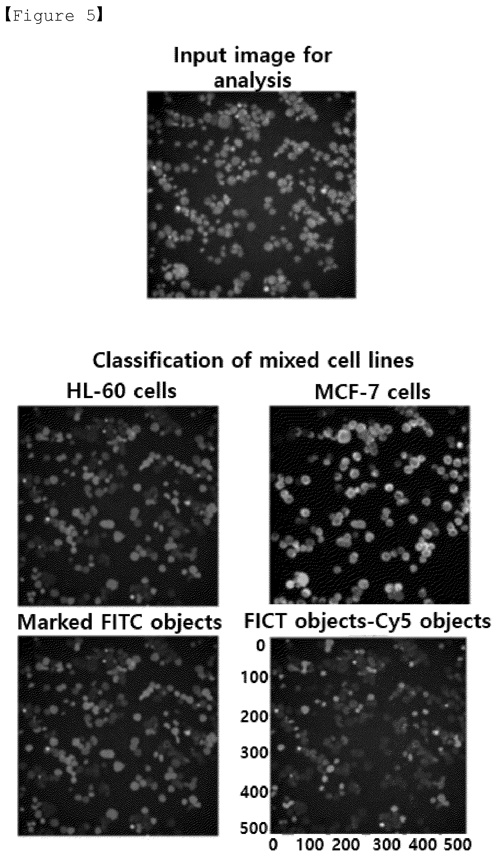

Accordingly, in the present invention, as shown in FIG. 5, by excluding the cancer cell detection results of the Cy5 channel from the white blood cell detection results of the FITC channel image, only actual white blood cells remain in the FITC channel image.

### Step of extracting targets and providing spatial information (S14)

Depending on the type of target, a fluorescence channel image to be used to acquire spatial information of the target is selected, and the location, size, shape, etc. of each object existing in the corresponding fluorescence channel are converted into spatial information of each target and provided to a biochemical molecule isolation device 200.

That is, if the target is a white blood cell, spatial information of the targets is acquired based on the white blood cell detection result of the FITC channel image and provided to the biochemical molecule isolation device 200, and if the target is a cancer cell, spatial information of the targets is acquired based on the cancer cell detection result of the Cy5 channel image and provided to the biochemical molecule isolation device 200.

Then, the biochemical molecule isolation device 200 adjusts the laser irradiation size and location depending on the spatial information of the targets to isolate the targets existing in the tissue sample, and then isolates and retrieves them through a plurality of wells.

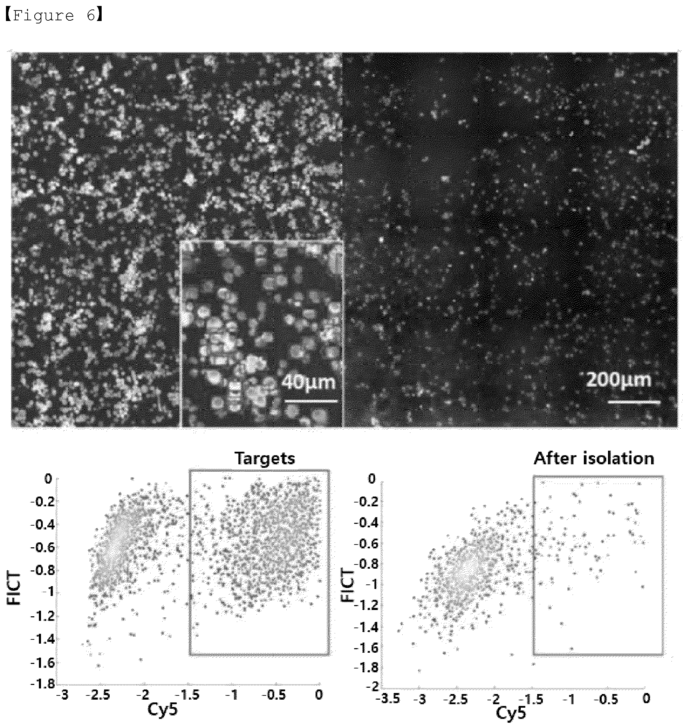

FIG. 6 is a drawing showing the result of target isolation by a biochemical molecule isolation device, and by referring to this, it can be confirmed that only the targets (e.g., cancer cells) were selectively isolated and retrieved from the tissue sample.

In addition, there is a characteristic that the size of the fluorescence image that may be acquired through a single imaging operation is very small compared to the overall size of the tissue sample.

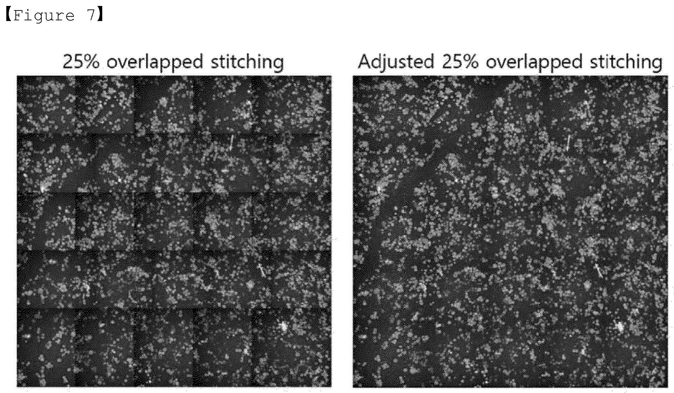

Accordingly, in the present invention, by acquiring a plurality of fluorescence images in a way of continuously scanning the entire tissue sample and then stitching them into a single image as shown in FIG. 7, fluorescence images of a wide area corresponding to the entire tissue sample may be acquired. However, considering that the illumination may change when acquiring images, the illumination homogenization operation is performed together with the image stitching operation.

In addition, by performing target detection operation using a wide area of fluorescence image, a wider range may be analyzed more quickly.

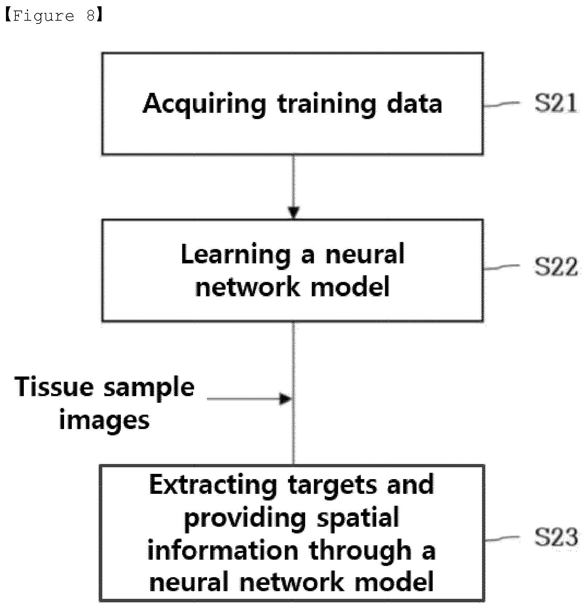

FIGS. 8 to 11 are drawings for explaining the method for detecting a target based on a neural network according to another embodiment of the present invention.

As shown in FIG. 8, the method for acquiring the neural network-based spatial information of the present invention is comprised of a step of acquiring training data (S21), a step of learning a neural network model (S22), and a step of analyzing a tissue image based on the neural network model (S23).

### Step of acquiring training data (S21)

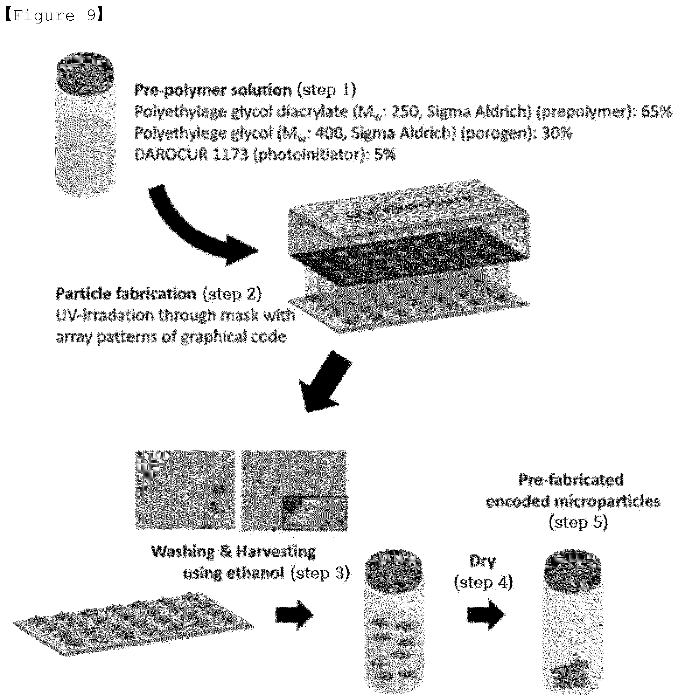

First, a plurality of microparticles corresponding to the target are generated. In this case, the microparticles may be acquired through, but are not limited to, a process of creating a liquid mixture of a pre-polymer and a photoinitator (step 1), a process of filling a tube array with the liquid mixture of the pre-polymer and the photoinitator, then covering it with a photomask having homogeneous codes, and then irradiating it with ultraviolet rays to fabricate homogeneous microparticles (step 2), a process of curing the microparticles and then removing them from the tube array (step 3), a process of washing the microparticles (step 4), and a process of harvesting the washed microparticles (S21-5), as shown in FIG. 9.

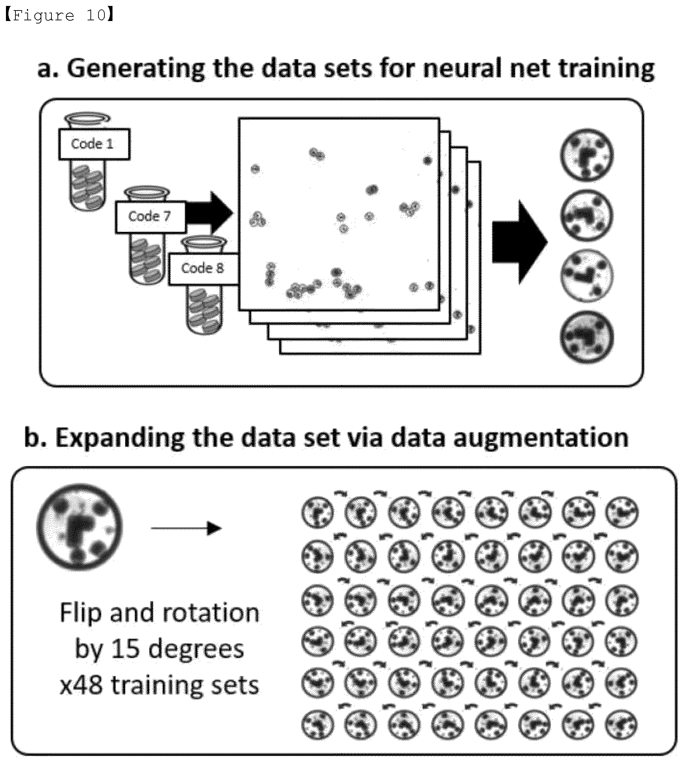

In addition, the fabricated microparticles are irregularly dispersed on a glass slide and then photographed to acquire a plurality of sample images. Each of the plurality of sample images is flipped and rotated to augment the number of sample images to be used for generating training data, as shown in FIG. 10.

Based on a plurality number of data-augmented sample images, a large amount of training data having the sample image as an input condition and the target matching probability as an output condition is generated. In this case, the training data is used to be, but not limited to, 70% of the tissue images as a training set, 15% as a validation set, and 15% as a test set.

### Steps of learning a neural network model (S22)

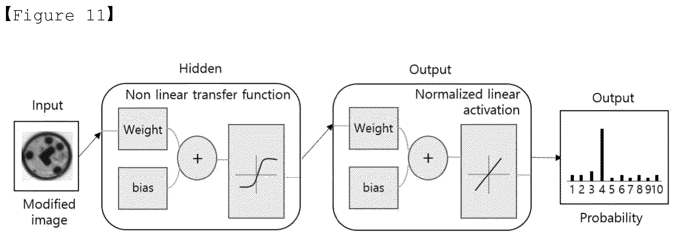

The neural network model of the present invention may be implemented by a DNN (Deep Neural Network), a CNN (Convolution Neural Network), an RNN (Recurrent Neural Network), etc., and has an input layer, a hidden layer, and an output layer as shown in FIG. 11, and learns the target detection probability corresponding to the input image through a plurality of training data.

In addition, the prediction accuracy is calculated by comparing the sorting results using the neural network model with the predicted results, and if the prediction accuracy exceeds the desired value, the neural network learning is terminated.

### Step of extracting targets and providing spatial information through a neural network model (S23)

When the neural network model has been learned, if a tissue sample is mounted on an ITO glass slide and then placed on the sample stage 221 of a biochemical molecule isolation device 200, a general image of the tissue sample is acquired through a CCD camera 211 while light is shone on the tissue sample through a target light source 214 and a light source 215.

In addition, image regions, in which the matching probability between the tissue image and the target through a learned neural network model is a preset value or greater, are detected, and the location, size, shape, etc. of each of these image regions are acquired and provided as spatial information of the target.

In the above description, training data is generated by directly fabricating microparticles corresponding to the target, but if necessary, target images may be acquired from medical images photographed through various medical imaging devices, and training data may be generated through these.

In addition, target images may be acquired through multi-channel fluorescence images, and training data may be generated through these.

However, in such cases, it would be desirable to enable a neural network-based target detection operation to be performed in response to a request from a worker, under the assumption that the tissue sample has been stained, without a process of checking whether the tissue sample has been stained.

Although the preferred embodiments of the present invention have been shown and described above, it is natural that the present invention is not limited to the specific embodiments described above and various modifications may be made by those skilled in the art to which the present invention pertains without departing from the gist of the present invention as claimed in the claims, and such modifications should not be understood individually from the technical spirit or prospect of the present invention.

## Claims

1. A method for isolating biochemical molecules in a system for isolating biochemical molecules, comprising the steps of:
imaging a tissue sample to acquire a tissue image in the form of any one of a multi-channel fluorescence image and a visible light image;
obtaining and analyzing detection results for each channel from the tissue image to extract spatial information of a target, or analyzing the tissue image through a learned neural network model to predict spatial information of a target; and
adjusting the size and location of an isolation area in a biochemical molecule isolation device based on the spatial information of the target, and then physically isolating and retrieving the target from the tissue sample.

2. The method for isolating biochemical molecules according to claim 1, wherein the target is any one of a cell, a group of two or more cells, a micro-region containing a cell, ribonucleic acid (RNA), deoxyribonucleic acid (DNA), an epigenome, an epitranscriptome, a metabolite, a cell organelle, a B cell receptor (BCR), a T cell receptor (TCR), an extracellular membrane protein, an extracellular vesicle, a carrier containing a biochemical molecule, and a hydrogel containing a biochemical molecule.

3. The method for isolating biochemical molecules according to claim 1, wherein the spatial information of the target may include at least one of information on location, size, shape, density, and staining pattern.

4. The method for isolating biochemical molecules according to claim 1, wherein the extracting of the spatial information of the target includes the steps of:
acquiring a multi-channel fluorescence image;
isolating fluorescence channels and then performing an object detection operation;
comparing and analyzing object detection results for each fluorescence channel to filter the object detection results; and
selecting at least one or more of the fluorescence channels in consideration of the type of a target, and extracting and providing the spatial information of the target from the object detection results of a selected fluorescence channel.

5. The method for automatically isolating cells according to claim 4, wherein, when the target is a cell, the filtering includes refining and extracting only the spatial information of the target, by acquiring object detection results for each fluorescence channel based on at least one of location, shape, size, density and fluorescence intensity and then filtering out duplicate objects and noise objects from the object detection result for each fluorescence channel.

6. The method for isolating biochemical molecules according to claim 1, wherein the neural network model is pre-trained on data mapping tissue images to target detection results, by repeatedly training on multiple datasets having a sample image as an input condition and a target detection probability as an output condition.

7. The method for automatically isolating cells according to claim 1, wherein the physically isolating and retrieving of the target includes physically isolating the target through any one method of isolation using lasers, mechanical isolation through microdissection, isolation using high-frequency ultrasound, chemical isolation through the addition of specific chemicals or enzymes, biological isolation using antibodies specific to specific cell surface antigens, optical isolation using light, and thermal ablation using high temperatures.

8. A method for spatially designating biochemical molecules in a system for isolating biochemical molecules, comprising the steps of:
imaging a tissue sample to acquire a tissue image in the form of any one of a multi-channel fluorescence image and a visible light image; and
obtaining and analyzing detection results for each channel from the tissue image to extract spatial information of a target, or analyzing the tissue image through a learned neural network model to acquire spatial information of a target, and then providing it to an external party.

9. The method for isolating biochemical molecules according to claim 8, wherein the target is any one of a cell, a group of two or more cells, a micro-region containing a cell, ribonucleic acid (RNA), deoxyribonucleic acid (DNA), an epigenome, an epitranscriptome, a metabolite, a cell organelle, a B cell receptor (BCR), a T cell receptor (TCR), an extracellular membrane protein, an extracellular vesicle, a carrier containing a biochemical molecule, and a hydrogel containing a biochemical molecule.

10. The method for isolating biochemical molecules according to claim 8, wherein the spatial information of the target may include at least one of information on location, size, shape, density, and staining pattern.

11. The method for isolating biochemical molecules according to claim 8, wherein the extracting of the spatial information of the target includes the steps of:
acquiring a multi-channel fluorescence image;
isolating fluorescence channels and then performing an object detection operation;
comparing and analyzing object detection results for each fluorescence channel to filter the object detection results; and
selecting at least one or more of the fluorescence channels in consideration of the type of a target, and extracting and providing the spatial information of the target from the object detection results of a selected fluorescence channel.

12. The method for isolating biochemical molecules according to claim 8, wherein the neural network model is pre-trained on data mapping tissue images to target detection results, by repeatedly training on multiple datasets having a sample image as an input condition and a target detection probability as an output condition.

13. A method for providing spatial information of a target for a system for automatically isolating biochemical molecules, comprising the steps of:
acquiring a multi-channel fluorescence image;
isolating fluorescence channels and then performing an object detection operation;
comparing and analyzing object detection results for each fluorescence channel to filter out duplicate objects and noise objects; and
selecting any one of the fluorescence channels in consideration of the type of a target and extracting the spatial information of the target from the object detection results of a selected fluorescence channel to provide it to an external party.

14. A method for providing spatial information of a target for a system for automatically isolating biochemical molecules, comprising the steps of:
pre-learning a neural network model through a plurality of training data having a sample image as an input condition and a target matching probability as an output condition;
imaging a tissue sample to acquire a tissue image in the form of any one of a multi-channel fluorescence image and a visible light image; and
analyzing the tissue image through the learned neural network model to extract spatial information of a target and providing it to an external party.

15. A system for automatically isolating biochemical molecules, comprising:
a target automatic detection device for inputting a tissue image acquired in the form of any one of a multi-channel fluorescence image and a visible light image, and obtaining and analyzing detection results for each channel from the tissue image to extract spatial information of a target, or analyzing the tissue image through a learned neural network model to acquire spatial information of the target; and
a biochemical molecule isolation device for acquiring the tissue image to provide it to the target automatic detection device, then adjusting the size and location of an isolation area based on the spatial information of the target fed back from the target automatic detection device, and then physically isolating and retrieving the target from a tissue sample.

16. A computer-readable recording medium on which a program for executing the method for automatically isolating cells as described in any one of claims 1 to 9.
